# EUROPEAN PATENT APPLICATION

(11) **EP 1 103 265 A1**
(43) Date of publication of application: **30.05.2001**
(21) Application number: 99203815.8
(22) Date of filing: 15.11.1999
(51) Int. Cl.: A61K 35/14, C12N 5/06, C12N 5/08, A61P 35/00, A61P 37/00

(54) **T-cells specific for polymorphic proteins on malignant cells**

(71) Applicant: Leids Universitair Medisch Centrum, 2333 ZA Leiden (NL)
(72) Inventor: FALKENBURG, Johan H. F., 2341 ET Oegstgeest (NL)
(74) Representative: Ottevangers, Sietse Ulbe

(57) **Abstract**

One aspect of the invention capatilizes on the fact that most genes are present in two copies in a cell and the fact that there exist polymorphic regions in at least some proteins. Polymorphic proteins that are expressed by tumor cells and that further show a limited tissue distribution are used to target immune therapies against the tumor cells. The invention provides among others, means and methods for killing such tumor cells and the generation of specific T-cells.

## Description

The invention relates to the field of medicine. In particular, the invention relates to the field of immunotherapy.

Cancer is a common cause of death in the human population. In spite of intensive efforts to develop cures for cancer, progress has been slow. Current methods for combating cancer rely on surgery to remove the cancer physically, on chemotherapy and on radiotherapy. Although these methods have proven to be very valuable in combating cancer, the methods are by far not able to cure all cancer patients. Novel methods for combating cancer are clearly desired.

A very promising new approach to the struggle against cancer is immunotherapy. The basic idea behind this approach is to utilize in some way the unique properties of the immune system to generate an immune response against the cancer cells in the patient. For long this approach has focused on the identification of tumor specific antigens which are as the word says only present on the tumor cells and can thereby form a specific target for directing an immune response. The specificity is desired to control the immune response against the cancer in such a way as to prevent inadvertent destruction of other tissue in the patient.

Many different tumor specific antigens have been discovered and immunotherapy approaches to combat cancer expressing such tumor specific antigens have been developed. However, a problem with this approach is that very many tumors do not express truly tumor specific antigens. Some tumor specific antigens are not very immunogenic causing the immunotherapy to be not very effective. Furthermore, frequently a so-called tumor specific antigen is in fact only over expressed in the tumor cells and although to a lesser extent, also expressed on healthy cells resulting in an immunotherapy that destroys many more cells than only the tumor cells.

There is therefore a need in the field to identify novel approaches toward immunotherapy. On the one hand there is a need for a wider spectrum of targets for the immunotherapy than presently available. On the other hand there is a need for a completely alternative approach for the development of the immunotherapy.

An object of the present invention is to provide more targets for directing an immune response against in the development of immunotherapy.

Another object of the present invention is to provide a new class of immunotherapies.

By far the most cancer cells originate from a single cell in an individual. Within that cell, the crucial mutation(s) occurred that formed the first step(s) in the malignancy process. Just as in all other cells, most genes are present in two copies in that first cell. One gene is derived from the father of the individual and one is derived from the mother of the individual. The most important exception, of course, being the genes lying on the X or the Y chromosome, in case the individual is a man.

In its simplest form a gene encodes one protein. Due to various mechanisms, however, various variants of a single gene may be present within one species. These variants encode proteins comprising similar functions, in spite of the fact that the amino acid sequence between the different variants differs. The difference in the amino acid sequence is very often restricted to one or more regions of the protein. In the present invention these regions are termed polymorphic regions. The polymorphic regions may differ in proteins encoded by genes derived from the father or mother.

One aspect of the present invention capitalizes on the fact that most genes are present in two copies in a cell and the fact that there exist polymorphic regions in at least some proteins. The present invention relates to polymorphic proteins which are expressed by the tumor cells, and show a limited tissue distribution among normal cells of the individual with the tumor. The tissue distribution of said proteins can be limited to tissues that are not essential to the patient, or that can be replaced by healthy tissues for instance by replacement by transplantation of the tissue derived from a donor (allogeneic transplantation) or by temporarily removing the tissue from the patient (autologous transplantation). In addition, tissue distribution of said proteins may be limited to tissues of which destruction of part of the tissue is permissible.

More specifically, the present invention relates to proteins of which one of the two polymorphic variants within one cell can be removed or inactivated resulting in expression of only one variant of the gene within one cell. Preferably, the inactivation of the gene is a randomly occurring process taking place prior to, or during malignant development of the precursor cell of the tumor, and is persistent in the progeny of the malignant cell.

The polymorphic part of the protein must be immunogenic, i.e. must have the property that it can be recognized by an immune system. Preferably, the polymorphic part of the protein contains a peptide sequence that can be recognized in the context of a major histocompatibility complex molecule by T cells via the T cell receptor (TCR).

Alternatively, the polymorphism of the genes expressed in the tumor cells as compared to some of the normal cells in the normal tissue may be due to inactivation of one of the genes with comparable function. For example, during B cell differentiation, the light chain of the immunoglobulin chain consists within one B cell of either a kappa or a lambda chain. Cells from one particular B cell tumor express exclusively only kappa or lambda chains, whereas similarly the normal cells randomly express either a kappa or a lambda chain. Therefore, an immune response against peptides derived from the light chain that is expressed in the tumor will affect all tumor cells, but only part of the normal B cells, i.e. only the B cell expressing the same light chains as the tumor cell.

In summary, the present invention relates to proteins which have the following properties:
1) The protein is a polymorphic protein or comprises a polymorphic region that can be recognized by an immune system.
2) The protein is expressed in at least part of the tumor cells.
3) The protein shows a limited tissue distribution
4) The protein can be randomly removed or inactivated in the tumor (precursor) cells and in the tissues in which the gene is normally expressed, and this inactivation is persistent in at least part of the progeny of the malignant tumor (precursor) cells.

If the protein is expressed in normal tissues that can be removed or replaced by donor cells, both homozygous and heterozygous expression of the protein is permissible. In these cases an immune reaction against the polymorphic part of the protein that is expressed in the normal tissues will result in destruction of both malignant cells and the normal tissue, which may be replaced by donor tissue not expressing the patient-specific polymorphism.

Replacement of the normal tissue expressing the specific protein may not be necessary in case of a expression of a gene in a heterozygous individual in which one of the two polymorphic variants of the gene within normal and tumor cells one cell was removed or inactivated resulting in expression of only one variant of the gene within one cell. If an immune reaction is generated against the polymorphic variant expressed in the tumor cells, only that part of the normal tissue will be affected that expresses the same polymorphic variant as the tumor cells, whereas the other part of the normal tissue will not be affected.

In one aspect the invention provides a method for killing a malignant cell of an individual, wherein said malignant cell ultimately originated from one cell in said individual, wherein said one cell was a member of a group of cells with a limited distribution in said individual, said method comprising contacting said malignant cell with a T-cell, said T-cell comprising a T-cell receptor capable of recognizing a peptide/major histocompatibility complex molecule combination present on said malignant cell and present on a number cells of said group of cells and wherein said peptide is derived from a polymorphic protein essentially specifically expressed in said group of cells. Preferably, said group of cells comprises cells of the hemopoietic system, more preferably, B-cells.

Apart from the specific characteristics of the T-cell receptor said T cell can be any T cell capable of killing said target cell as long as at least part of the recognition of said target cell is achieved through said specific T cell receptor. Preferably, said T cell is a cytotoxic T cell. Said T-cell may be derived from a non-related donor individual. However, preferably said T-cell is derived from said individual or a functional equivalent thereof. A functional equivalent of said individual with respect to the origin of the T cell is an HLA-matched donor and more preferably, an HLA identical donor, and most preferably an HLA identical family related donor.

Said malignant cell ultimately originated from one cell in said individual. However, the method of the invention of course also works to kill another malignant cell originating from another one cell, when said another one cell was a member of the same group of cells with a limited distribution in said individual as said one cell in said individual, provided that said another one cell expresses said peptide/MHC molecule combination. Although said malignant cell ultimately originated from one cell in said individual, said malignant cell, of course, can have undergone many cell divisions prior to said killing. Thus said malignant cell may in fact be part of a group of malignant cells all originating from said one cell in said individual. Said malignant cell, though ultimately originating from one cell in said individual may have undergone additional genetic and/or phenotypic changes from the moment of origination.

Said MHC molecule can be an MHC class I and/or class II molecule. Preferably, said MHC molecule is an MHC class I molecule.
Said number of cells can comprise the entire number of cells present in said group of cells. Of course, if such is the case and said individual cannot do without essentially all the cells in said group of cells then it is advisable to transplant said individual with cells of said group of cells obtained from a donor. Said cells of said group of cells obtained from said donor, in that case preferably, should not all comprise said peptide/MHC molecule combination, since if this would be the case all the transplanted cells also provided a target for said T-cell. The combination of the peptide with the MHC molecule provides the discriminating property that is recognized by said T-cell. Thus when said T-cell is an allogeneic T-cell, non-polymorphic parts of, by way of non-limiting example, an immunoglobulin can be used for immunotherapeutic approaches in the context of allogeneic stem cell transplantation, if donor and recipient despair for an HLA-molecule. If a non-polymorphic part of the immunoglobulin is presented in an HLA-molecule that differs between donor and recipient, this will lead to a B cell specific antigen that can be recognized by T cells from the donor leading to eradication of all B cells from the patient including the malignant B cells. Cells not belonging to the B cell lineage will not be destructed since they do not express the immunoglobulin gene. Similarly, donor derived B cells will not be eliminated since the donor cells do not exhibit the appropriate MHC-molecule necessary for the recognition of the specific immunoglobulin/MHC-complex by donor cells. Therefore, if allogeneic stem cell transplantation is performed using an HLA-mismatched donor, T cell responses against peptides derived form the immunoglobulin gene presented in MHC-class I molecules not present on donor cells will lead to an elimination of the malignant cells including normal cells from the patient, not effecting donor hematopoiesis.

Preferably, said number of cells comprises a subset of the cells of said group of cells, wherein said subset does not comprise the essentially all the cells of said group of cells in said individual. When said number of cells comprises a subset of said group of cells, at least some cells of said group of cells are spared from being a target for said T cell and therefore are able to persist in said individual in the presence of said T-cell. The spared cells can at least in part complement the function of said group of cells in said individual. The spared cells can also take over the entire function of said group of cells in said individual. They can do so, for instance but not limited to, by dividing until their number approximates the number of the group of cells. Preferably, said number of cells comprises between 20% and 80% of all the cells present in said group of cells. More preferably said number of cells comprises between 30% and 70% of all the cells present in said group of cells. Most preferably, said number of cells comprises between 40% and 60% of all the cells present in said group of cells.

In a preferred embodiment said peptide comprises a polymorphic part of said protein. Preferably, said individual is heterozygous for a gene encoding said polymorphic protein and at least part of the cells expressing said polymorphic protein comprise essentially only polymorphic protein derived from either the maternally or the paternally derived gene. When said peptide is derived from the protein encoded by the maternally derived gene, cells expressing essentially only the paternally derived gene will not be a target for said T cell in the case said peptide comprises a polymorphic part of said protein.

Preferably, said maternally or said paternally derived gene, at a time point prior to the origination of said malignant cell, is inactivated. Said inactivation may occur through any method. Non-limiting examples are paternal imprinting, maternal imprinting, random inactivation through methylation or other methods. Preferably, said inactivation is achieved through X chromosome inactivation in an individual comprising at least two X chromosomes, such as a woman.

In one embodiment of the invention said polymorphic protein is expressed in at least part of the cells of the hemopoietic system. Preferably said polymorphic protein is expressed in B cells. Preferably, said polymorphic protein comprises an immunoglobulin.

B cell malignancies usually express a single (clonal) immunoglobulin gene. Previously it has been suggested and investigated whether the specific antigen-recognizing part of the immunoglobulin (the idiotype) can be used as target structure for humoral and cellular immunotherapeutic approaches. Antibody treatment or vaccination protocols in patients suffering from B cell malignancies using the idiotype as target structure have been explored and although the efficacy of this response has not been clearly proven, the feasibility of the approach has been demonstrated. The theoretical basis of this approach is the unique feature of the idiotype of the malignancy within the B cell population of the patient. This approach however has various severe limitations. First, for each individual patient a specific response has to be generated. Secondly, for each individual idiotype it has to be demonstrated that this idiotype is presented appropriately on the cell membrane or in the context of MHC-molecules, and it can be recognized by the effector mechanism. This individual approach leads to a major logistic complexity and may impair the general applicability.

Apart from the idiotype parts of the immunoglobulin protein also other parts of the immunoglobulin proteins show differences between individuals. This polymorphism is called the allotype of the immunoglobulin. The allotypic polymorphisms represent amino-acid differences, usually not relevant for the function of the immunoglobulin, which are mutations of the gene in the general population. The frequency of some of the allotypes in the general populations are represented in Table 1. There are approximately 28 human immunoglobulin allotypes known thus far. These allotypes are defined at the protein level. Table 2 contains amino-acid differences between some of these allotypes.

If a B cell malignancy in the patient is expressing a certain immunoglobulin allotype which is not expressed in donor cells, an immune response against this allotype may eliminate the malignant cells from the patients including all normal B cells expressing the same allotype from the same patient. However, if donor B cell hematopoiesis is present in the patient, this allo- immune response will not lead to B cell deficiency. In particular, if peptides derived from the immunoglobulin proteins containing one of the polymorphic parts are presented and expressed in HLA-class I or class II molecules on the cell membrane of the B cells from the patient, T cells derived from the donor can exhibit a cellular immune response against the malignant cells leading to complete eradication of these cells. In conclusion, polymorphic peptides derived from the allotypic part of the immunoglobulin genes can be used as targets for immunotherapy in the context of allogeneic immunotherapy.

In one embodiment of the invention, peptides derived from allotypic parts of the immunoglobulin gene can function as targets for immunotherapy in, for instance, the autologous situation. During development, B cells will usually express only one immunoglobulin gene from one of the two chromosomes from paternal or maternal origin. Since this is a more or less a random process, the total B cell population arising from stem cells in an individual will express both immunoglobulin genes from the parental genes. In patients that are heterozygous for allotypes of the immunoglobulin gene, the progeny of one particular B cell will express only one of the two allotypes whereas the general population will of course contain both allotypes. Since the B cell malignancy is a clonal abnormality, in most patients with B cell malignancies the neoplastic cell will express only one of the two allotypes. Approximately half of the normal B cells will express the same allotype as the malignancy whereas the other half of the T cells will express the allotype which is not present on the malignant cells. Therefore, if an immune response is generated against the allotype specific for the malignancy, treatment with the humoral or cellular effector cells eliminates the malignant cells and only those normal B cells that express the same allotype as the malignant cell. All cells expressing the other allotype will be saved. Therefore, in heterozygous patients a T cell response against B malignancy associated allotypic gene products can be used in the autologous setting for the treatment of hematological B cell malignancies without impairing the B cells not expressing this allotype. Since in B cell development the use of one of the two chromosomes is probably a random process, it is expected that a normal B cell repertoire will be still present after the treatment and therefore this treatment will not lead to B cell or immunoglobulin deficiency.

In another embodiment of the invention proteins derived from genes that are randomly inactivated during development function as targets for autologous immunotherapy in malignancies.

Similar to the approach described in the previous embodiment, other proteins derived from polymorphic genes that are randomly inactivated during development can be target structures for immunotherapy of malignancies. For instance, in women, one of the two X-chromosomes is randomly inactivated during development. Since malignancies are clonal disorders, the malignancy will express genes present on only one of the two parental chromosomes. In contrast, the non-malignant counterpart of the malignant cells will randomly express one of the two genes from the X-chromosomes. If the gene of interest is restricted to a specific cell type, an immune response against this polymorphic gene will eliminate the malignant cells and half of the normal counterpart. Since one of the limitations of the use of immunotherapy in the autologous setting is usually the destruction of the normal counterpart of the malignant cell, this approach may lead to destruction of only half of the normal counterpart. In many cases the normal tissues have sufficient redundancy or repair mechanisms to allow sufficient function of the cells, tissues or organs. For instance, in hematological malignancies, if half of the stem cells will be eliminated, normal hematopoiesis will be left as can be demonstrated with the ability of only a small proportions of the stem cells to restore an allogeneic individual in allogeneic stem cell transplantation. Of course, the applicability of this approach is predominantly restricted to genes that are heterozygous on the two chromosomes of which one is inactivated.

In one aspect of the invention an additional level of safety is build into said T-cell. Of course the use of said T cell in said individual is safe considering the remarkable specificity of said T-cell receptor. However, in a preferred embodiment of the invention an additional level of control is build in through providing said T-cell with a system allowing said T-cell to be killed in response to a signal. In one embodiment said system is a so-called suicide system. A non-limiting example of such a system is a nucleic acid encoding herpes simplex virus (HSV) thymidine kinase (TK) or a functional part, derivative and/or analogue thereof. Wherein said signal is provided by providing said individual with gancyclovir or functional equivalent, derivative and/or analogue thereof. When said T-cell has been provided with said nucleic acid encoding HSV TK and said nucleic acid is epxressed in said T cell, providing said T cell with an appropriate amount of gancyclovir will lead to a conversion of said gancyclovir by said HSV-TK into a toxic compound that, when present in sufficient amounts, kills the T cell. Similar suicide systems have been described and can be used by a person skilled in the art to generate the system of this preferred embodiment.

In another aspect the invention provides a method for generating a T-cell comprising a T-cell receptor capable of recognizing a peptide/major histocompatibility complex molecule combination present on a malignant cell of an individual, wherein said malignant cell ultimately originated from one cell in said individual, wherein said one cell was a member of a group of cells with a limited distribution in said individual, and wherein said peptide is present on a number of cells of said group of cells, wherein said peptide is derived from a polymorphic protein essentially specifically expressed in said group of cells, said method comprising contacting a dendritic cell comprising said peptide/histocompatibility complex molecule combination, with a collection of T-cells, co-culturing said T-cells and said dendritic cell, culturing T-cells derived from said co-culturing, cloning said T-cells and determining the specificity of individual clones of said T-cells and selecting the T-cell clone comprising said T-cell receptor.

In one embodiment, said T cell clone is expanded. With expanded is meant increase in the number of cells. Preferably, at least part of said expansion is performed *in vitro.* This can be achieved through culturing said T-cell clone in the presence of said peptide in the presence of appropriate culture medium. Said culture medium preferably, comprises a factor supporting the expansion of said T cell clone. Preferably, said factor comprises IL-2, IL-12 and/or a combination thereof.

The expansion can of course be combined with the method for the generation of said T cell, preferably in a step comprising the culture, cloning, determining the specificity and/or the selection of said T cell. An advantage of combining said expansion with said generation method is that through this combination the clone to be generated can be at least in part specifically be expanded, due to the use of said peptide.

In another embodiment the invention provides a T-cell comprising a T-cell receptor capable of recognizing a peptide/major histocompatibility complex molecule combination present on a malignant cell of an individual, wherein said malignant cell ultimately originated from one cell in said individual, wherein said one cell was a member of a group of cells with a limited distribution in said individual, and wherein said peptide is present on a number of cells of said group of cells, wherein said peptide is derived from a polymorphic protein essentially specifically expressed in said group of cells.

In another aspect the invention provides a T-cell of the invention for use in a method of the invention.

In yet another aspect the invention provides a pharmaceutical composition comprising a T-cell according to the invention.

In still another aspect the invention provides the use of a T-cell of the invention, for the preparation of a medicament for the treatment of a malignancy. Preferably, said malignancy is a B-cell malignancy.

### EXAMPLES

### The generation of CTL response against peptides specific for the allotypic (polymorphic) part of the immunoglobulin heavy or light chain.

a. Some of the polymorphisms in the immunoglobulin heavy and light chains and the frequencies of these polymorphisms in the population are indicated in Table I.

**Table I**

| Frequency of the different allotypes in the general population | | |
|---|---|---|
| **IgH/IgL** | **Allotype** | **Haplotype Frequency** |
| Kappa | 3 / 1,2 | Km₃ : 83 % |
| | | Km_{1,2} : 17 % |
| IgG₁m | z / f | IgG₁m z : 28.9 % |
| | | IgG₁m f : 69.9 % |
| IgG₁m | a / -a | IgG₁m a : 28.9 % |
| | | IgG₁m -a : 69.9 % |
| IgG₁m | x / -x | IgG₁m x : 28.9 % |
| | | IgG₁m -x : 69.9 % |
| IgG₂m | n / -n | IgG₂m n : 28.9 % |
| | | IgG₂m -n : 69.9 % |
| IgG₃m | b / g | IgG₃m b : 28.9 % |
| | | IgG₃m g : 69.9 % |
| IgA₂ | 1 / 2 | IgA₂m 1 : 98.2 % |
| | | IgA₂m 2 : 1.8 % |

Immunoglobulin allotypes are the polymorphic antigenic determinants of immunoglobulins (Ig). There are approximately 28 human Mendelian Ig allotypes, five of these, A2m, Em, G1m, G2m and G3m, are situated in chromosome 14 band q32 and one Km, is situated in chromosome 2 band p12. All these allotypes are defined at the protein level and are strictly distributed within their proper Ig class of Ig subclass. Many of them are precisely defined as to amino acid and codon substitutions as shown in Table II.

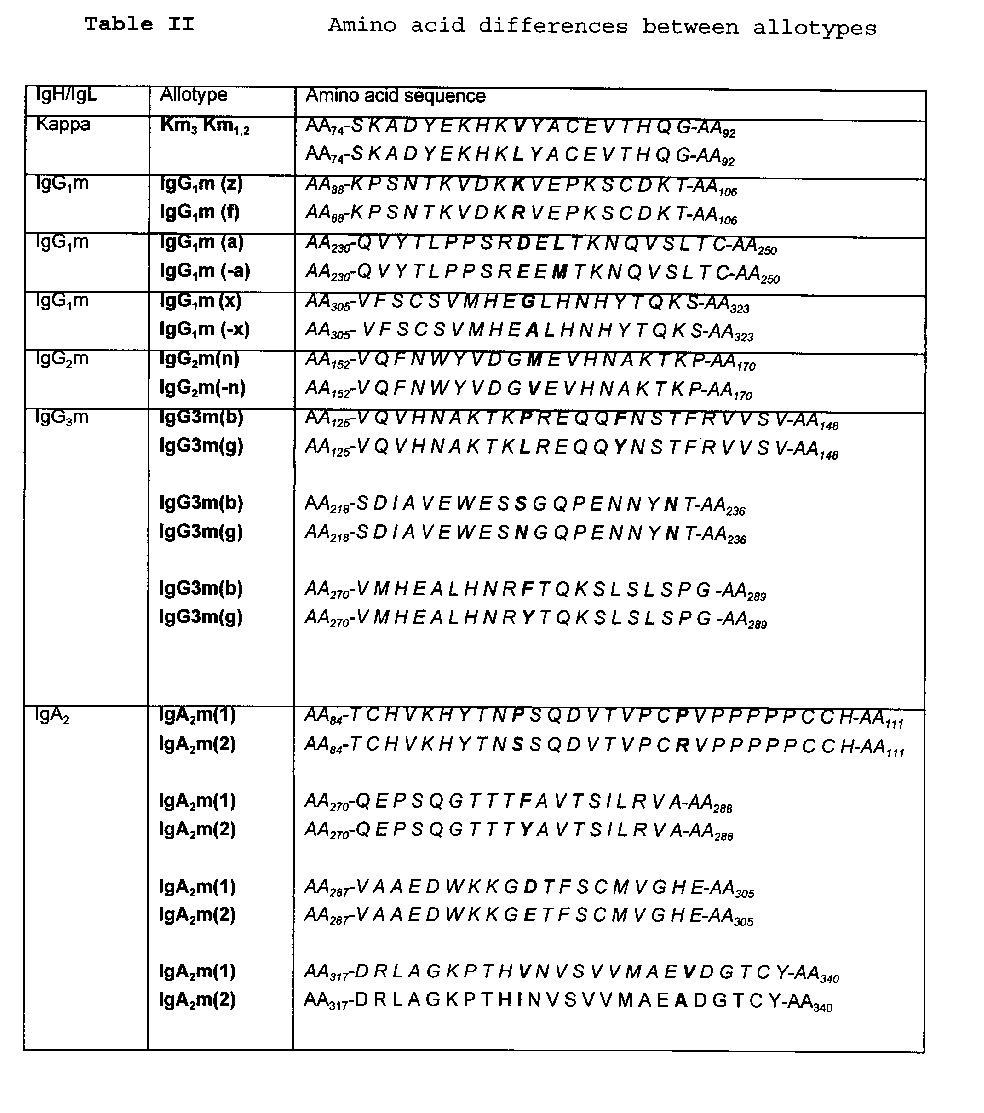

Malignant B cells should be screened on their Ig isotype expression either by FACS analysis or cytospin analysis (performed for diagnostic purposes at hematological centers). If the malignant B cell population expresses IgG₁₋₃, IgA₂ heavy chain or κ light chain, the specific allotype of the isotype should be determined. The allotype typing is performed using isolated genomic DNA and PCR analysis combined with restriction site analysis or DNA sequencing of the PCR products. If the patient is homozygote for the allotype of the malignancy no future determination of allotype is required. However if the patient is heterozygote for the specific allotype, the allotype of the malignant cells must be determined using RT-PCR performed on mRNA isolated from the malignant B cells.

b. Exploring the binding of polymorphic peptides derived from the immunoglobulin heavy or light chain in HLA-molecules and the generation of T cell responses against these peptides.

As mentioned above in (a), the differences between the allotypes are defined at the protein level and are strictly distributed within their proper Ig class of Ig subclass. To generate a peptide specific immune response the peptides containing the allotype difference should be presented in HLA class I or class II. To analyze whether a peptide is able to bind in the groove of the HLA complex the 20-30 mer peptides (containing the allotype difference) are analyzed on the presence of HLA binding motifs. Each peptide is analyzed for the known binding motifs within different HLA molecules using a computer program. Using this program it is possible to find the combination of peptide and HLA-molecule which is able to present the peptide in order to generate an immune response. T cell responses against the peptides are generated using either dendritic cells generated from donor CD14⁺ monocytes or CD34⁺ bone marrow cells. These DCs are pulsed with synthetic peptide and used as stimulator cells for the induction of the primary immune response. During the primary response the cells are cultured in the presence or absence of low levels of IL-2 and IL-12. On day 7 the CTL are stimulated with peptide pulsed CD14⁺ monocytes. One day after the restimulation 100 u/ml of IL-2 is added to the culture medium. Using this approach an allotype specific CTL response can be generated.

Alternatively, peptide specific T cells can be isolated from peripheral blood of donors using peptide/MHC tetrameric complexes. These tetrameric complexes specifically bind to T cells containing the T cell receptor specific for the MHC/peptide complex. Alternatively, peptide specific T cells can be isolated from blood using the gamma-Interferon secretion assay. In this assay antigen presenting cells loaded with specific peptides are co-cultured with autologous T cells. After 6-48 hours of culture gamma-Interferon producing responder cells are isolated and further expanded in the presence of specific peptide loaded antigen presenting cells. Both techniques have been shown to result in a high frequency of peptide specific T cells.

c. The analysis of potential and presentation of endogenously produced immunoglobulin peptides in the context of HLA class I and class II molecules.

One of the processes required for the presentation of a peptide in the groove of the HLA complex is endogenous cleavage of the protein by the proteasome.

### 1. Tranfection with mini-genes:

Processing and surface expression of the peptide/HLA complex can be analyzed using a Hela cell line transfected with the proper HLA molecule cotransfected with a mini-gene containing the allotype specific sequence. When the HLA-peptide complex is stable expressed in the Hela cell line, the cell line be recognized by the peptide derived CTL line. When the CTL recognizes the transfected Hela cell line TNF-α production can be measured in the supernatant. Analyzing several mini gene constructs, the allotype specific peptide presented in the context of the HLA complex can be elucidated.

### 2. Proteasomal cleavage of 20-30 oligomer peptides:

Using sequential isolation procedures it is possible to isolate proteasomes for cell lysate. Using these isolated proteasomes it is possible to study the proteasomeal cleavage of 20-30 oligomer petides containing the allotype sequence. The result of the proteasomal cleavage is analyzed using mass-spectrometry. Using this technique it may be possible to predict if an oligomer is cleaved is such a way that it is appropriately processed and presented in the groove of the HLA complex.

d. The analysis of the ability of allotypic specific CTL to effectively lyse chronic B cell malignancies.

After 3 cycles of restimulation the CTLs are tested for their cytolytic activity in a standard ⁵¹Cr release assay using donor derived EVB transformed B lymphocytes as targets pulsed with or without peptide and the allotype counterpart. When the CTL is able to lyse the EBV-LCL line pulsed with the peptide used the induction of the response and not the EBV LCL lines pulsed with the allotype counterpart, the cytolytic activity of the CTLs is tested against the malignant B cells. Furthermore the CTL will be analyzed for the reactivity with normal hematopoietic cells such as normal B cells and PHA stimulated lymphocytes from both donor and recipient to exclude non-specific reactivity.

## Claims

1. A method for killing a malignant cell of an individual, wherein said malignant cell ultimately originated from one cell in said individual, wherein said one cell was a member of a group of cells with a limited distribution in said individual, said method comprising contacting said malignant cell with a T-cell, said T-cell comprising a T-cell receptor capable of recognizing a peptide/major histocompatibility complex molecule combination present on said malignant cell and present on a number of cells of said group of cells and wherein said peptide is derived from a polymorphic protein essentially specifically expressed in said group of cells.

2. A method according to claim 1, wherein said number of cells comprises between 20% and 80% of all the cells present in said group of cells.

3. A method according to claim 2, wherein said number of cells comprises between 30% and 70% of all the cells present in said group of cells.

4. A method according to claim 3, wherein said number of cells comprises between 40% and 60% of all the cells present in said group of cells.

5. A method according to anyone of claims 1-4, wherein said T-cell is derived from said individual or a functional equivalent thereof.

6. A method according to anyone of claims 1-5, wherein said peptide comprises a polymorphic part of said protein.

7. A method according to anyone of claims 1-6, wherein said individual is heterozygous for a gene encoding said polymorphic protein and wherein at least part of the cells expressing said polymorphic protein comprise essentially only polymorphic protein derived from either the maternally or the paternally derived gene.

8. A method according to claim 7, wherein said the maternally or the paternally derived gene, at a time point prior to the origination of said malignant cell, is inactivated.

9. A method according to claim 8, wherein said is present on an X-chromosome.

10. A method according to anyone of claims 1-8, wherein said polymorphic protein is expressed in at least part of the cells of the hemopoietic system.

11. A method according to claim 10, wherein said polymorphic protein is expressed in B cells.

12. A method according to claim 10-11, wherein said polymorphic protein comprises an immunoglobulin.

13. A method according to anyone of claims 1-12, wherein said T-cell has been provided with a system allowing said T-cell to be killed in response to a signal.

14. A method for generating a T-cell comprising a T-cell receptor capable of recognizing a peptide/major histocompatibility complex molecule combination present on a malignant cell of an individual, wherein said malignant cell ultimately originated from one cell in said individual, wherein said one cell was a member of a group of cells with a limited distribution in said individual, and wherein said peptide is present on a number of cells of said group of cells, wherein said peptide is derived from a polymorphic protein essentially specifically expressed in said group of cells, said method comprising contacting a dendritic cell comprising said peptide/histocompatibility complex molecule combination, with a collection of T-cells, co-culturing said T-cells and said dendritic cell, culturing T-cells derived from said co-culturing, cloning said T-cells and determining the specificity of individual clones of said T-cells and selecting the T-cell clone comprising said T-cell receptor.

15. A T-cell comprising a T-cell receptor capable of recognizing a peptide/major histocompatibility complex molecule combination present on a malignant cell of an individual, wherein said malignant cell ultimately originated from one cell in said individual, wherein said one cell was a member of a group of cells with a limited distribution in said individual, and wherein said peptide is present on a number of cells of said group of cells, wherein said peptide is derived from a polymorphic protein essentially specifically expressed in said group of cells.

16. A T-cell according to claim 15, for use in a method according to anyone of claims 1-14.

17. A pharmaceutical composition comprising a T-cell according to claim 15 and/or claim 16.

18. Use of a T-cell according to claim 15 and/or claim 16, for the preparation of a medicament for the treatment of a malignancy.

19. A use according to claim 18, wherein said malignancy is a B-cell malignancy.
